# EUROPÄISCHE PATENTSCHRIFT

(11) **EP 2 934 607 B1**
(45) Veröffentlichungstag und Bekanntmachung des Hinweises auf die Patenterteilung: **02.03.2022**
(21) Anmeldenummer: 13820722.0
(22) Anmeldetag: 11.12.2013
(51) Int. Cl.: A61L 2/22, A61L 9/14, B05B 17/06

(54) **VERFAHREN UND VORRICHTUNG ZUM VERNEBELN VON WÄSSRIGEN POLYMERHALTIGEN BIOZIDEN MITTELS ULTRASCHALLTECHNOLOGIE IN EINEN RAUM**
METHOD AND DEVICE BY WHICH AQUEOUS POLYMER-CONTAINING BIOCIDES ARE ATOMIZED BY MEANS OF ULTRASOUND TECHNOLOGY INTO A ROOM
PROCÉDÉ ET DISPOSITIF POUR FORMER UN BROUILLARD DE BIOCIDES À TENEUR EN POLYMÈRE, AQUEUX, AU MOYEN D'UNE TECHNOLOGIE À ULTRASONS DANS UNE PIÈCE

(30) Priorität: 18.12.2012 AT 13062012
(43) Veröffentlichungstag der Anmeldung: 28.10.2015
(73) Patentinhaber: Rauchenschwandtner, Harald, 4851 Gampern (AT)
(72) Erfinder: Rauchenschwandtner, Harald, 4851 Gampern (AT); Gehmair, Reinhard, 4850 Timelkam (AT)
(74) Vertreter: Hübscher & Partner Patentanwälte GmbH
(86) Internationale Anmeldenummer: PCT/AT2013/050245
(87) Internationale Veröffentlichungsnummer: WO 2014/094019

(56) Entgegenhaltungen:
- EP-A1- 1 787 511
- WO-A1-2012/041910
- US-A- 4 612 777
- US-A1- 2011 284 656

## Beschreibung

### Technisches Gebiet

Die Erfindung bezieht sich auf ein Verfahren zum Ausbringen eines wässrigen, Guanidin enthaltenden Biozids in Form von Nebel mit einer durchschnittlichen Tröpfchengröße von kleiner 10 µm in einen Raum mittels Ultraschalltechnologie zur Desinfektion des Raumes.

### Stand der Technik

Die technische Aufgabe der Erfindung ist das Ausbringen von wässrigen Bioziden der eingangs geschilderten Art in einen Raum in Form von Nebel zur dreidimensionalen Desinfektion des Raums, der darin enthaltenen Raumluft und Oberflächen, wobei die Tröpfchengröße durchschnittlich unter 10 Mikrometer beträgt und das Biozid zerstäubt wird. Wässrige Biozide mit polymerhältigen Wirkstoffen wie beispielsweise CAS 374572-91-5, CAS 57028-96-3, CAS 27083-27-8, CAS 32289-58-0 (PHMB) etc, werden dazu mittels Pressluft und Düse im Raum zerstäubt.

Dazu ist es bekannt (WO 2012/041910 A1) eine wässrige, polymerhaltige Guanidinium- Verbindung mit einer Tröpfchengröße von überwiegend kleiner 10 µm zur Abtötung von Mikroorganismen in einen Raum auszubringen. Die Zerstäubung und das Ausbringen erfolgen dabei an einer Düse mit einem mit einer Frequenz von 18000 bis 30000 Hz schwingenden Schallgas. Im Gegensatz zu herkömmlichen Zerstäuberdüsen treffen das Schallgas und die wässrige, polymerhaltige Guanidiniumlösung erst kurz außerhalb der Düse aufeinander. Es genügt ein Flüssigkeitsdruck von 1-3 bar um die träge Lösung durch eine entsprechend große Bohrung zu fördern. Das mit einem mit einer Frequenz von 18000 bis 30000 Hz schwingende Schallgas zerreißt die wässrige, polymerhaltige Guanidinium- Verbindung in mikroskopisch kleine Teilchen. Das Zerstäuben erfolgt somit mit verhältnismäßig hohem Aufwand und bedingt durch das Schallgas unter einer nicht unerheblichen Geräuschkulisse.

Die US 2011/284658 A1 offenbart einen Aerosolgenerator mit einem Piezoelement, das eine Membrane zur Vernebelung einer Flüssigkeit anregt. Das auszubringende Fluid wird dabei einer löchrigen Membran von unten her zugeführt, unmittelbar an der Membran zerstäubt, und anschließend an die Umgebung abgegeben. Eine weitere bekannte Vorrichtung (EP 1 787 511 A1) umfasst eine Brennstoffzelle zum Betreiben einer Vorrichtung zum Ausbringen von Flüssigkeiten mit einer Ultraschallmembran und mit einem Gebläse. Allerdings ist die auszubringende Flüssigkeit wiederum in einem Behälter unterhalb der Membrane angeordnet und wird über eine Art Docht von unten an die Membrane herangeführt und an dieser unmittelbar zerstäubt.

Die Verwendung von Piezokeramikschwingern auf Ultraschallbasis bei der Zerstäubung von Wasser zur Luftbefeuchtung ist beispielsweise aus der US 4 612 777 A und der DE 197 13 496 A1 bekannt, die ein Verfahren und eine Vorrichtung für den Mineral- u. Kalkablagerungsfreien Betrieb eines piezoelektrischen Keramikschwingers, wie zur Erzeugung von Wassernebel benutzt, offenbart. Gemäß der Lehre dieser Druckschrift wird eine Transportflüssigkeit auf Glyzerin/Wasserbasis zwischen dem piezoelektrischen Keramikschwinger und einer Passivmembrane aus Plastik (Polyethylen) eingebracht, die den Aktuator vom Wasser trennt, das zur Zerstäubung ansteht.

### Darstellung der Erfindung

Ausgehend von einem Stand der Technik der vorgeschilderten Art liegt der Erfindung die Aufgabe zugrunde, ein Verfahren zum Ausbringen von wässrigen, Guanidin enthaltenden Biozids zur Raumdesinfektion dahingehend zu verbessern, dass das Ausbringen des wässrigen Biozids in Form von Nebel in einen Raum beispielsweise leise und energiesparsam möglich ist, wobei das Verfahren mit einer Vorrichtung ausgeführt werden kann, welche zudem insbesondere einfach zu transportieren sein soll.

Die Erfindung löste die gestellte Aufgabe dadurch, dass das Biozid zunächst mit wenigstens einer wenige Zentimeter unter der Fluidoberfläche schwingenden Ultraschallmembrane zerstäubt wird, wonach das zerstäubte, wässrige polymere, Guanidin enthaltende Desinfektionsmittel mit Luft als Trägermedium in den Raum ausgebracht wird.

Erfindungswesentlich ist es also, dass das wässrige Biozid mit polymerhaltigem Wirkstoff mit einer Ultraschallmembrane zerstäubt wird. Dadurch ist es möglich, die Vorrichtung praktisch geräuschlos zu betreiben, wobei zudem sichergestellt ist, dass nur ein verhältnismäßig geringer Energieeinsatz zur Zerstäubung erforderlich ist.

Das zerstäubte Biozid wird mit Luft als Trägermedium, mit mindestens einem Gebläse, in den Raum ausgebracht. Der Biozidnebel wird mittels vom Gebläse durch den Vernebelungsraum der Vernebelungsvorrichtung geförderter Luft aus der Vernebelungsvorrichtung zur Desinfektion und/oder zum Desodorieren ausgebracht.

Vorteilhaft ist es, wenn ein Guanidin enthaltendes polymerhaltiges Biozid mit der Ultraschallmembrane zerstäubt wird, wobei das Biozid vorzugsweise mit wenigstens einer Ultraschallmembrane, mit einer Frequenz von vorzugsweise >1Mhz, insbesondere von rund 1,7 MHz, aus Kunststoff, Quarz, Keramik oder Metall zerstäubt wird. Einmembrangeräte eignen sich beispielsweise für kleinere Räume, wie Kühlschränke, Kraftfahrzeuge u. dgl., wohingegen für größere Räume, wie Krankenzimmer, Küchen, Raucherzimmer, Wohnzimmer, Frachtcontainer, Industrielüftungsanlagen, Rettungswägen u. dgl. vorzugsweise Mehrmembrangeräte zum Einsatz kommen.

Ebenso ist es vorteilhaft, wenn das flüssige Biozid vor oder nach der Zerstäubung erwärmt wird. Ein Ausbringen des temperierten Biozids in der gewünschten Tröpfchengröße weist gegenüber dem aus dem Stand der Technik bekannten Pressluftverfahren viele Vorteile auf. Insbesondere kann ein feinerer Biozidnebel mit nur geringer Streuung in der Biozidtröpfchengröße erzeugt werden.

Das zerstäubte Biozid kann mit Hilfe von zumindest einem weiteren Gebläse im Raum verteilt werden. Gebläsevorrichtungen im Raum sorgen für eine homogene Verteilung der erzeugten Lösung. Bei Räumen mit bestehenden Gebläsen, wie Klimaanlagen, Lüftungsanlagen oder ähnlichen Leitungen bzw. Kanälen ist kein zusätzliches Gebläse notwendig.

Um sowohl die Vernebelungsleistung als auch die durchschnittliche Tröpfchengröße während wenigstens eines Behandlungszyklusses möglichst konstant halten zu können, wird vorgeschlagen, das Biozid aus einem Vorratsbehälter derart durch einen wenigstens eine Ultraschallmembrane aufweisenden Behälter mit einer Fördereinrichung derart umzuwälzen, dass der Pegel des wässrigen Biozides über der Membrane stets konstant gehalten wird. Ein Austritt von Tröpfchen mit einer zu großen Größe kann unterbunden werden, wenn das vernebelte Biozid mit dem Gebläse über einen Kamin ausgebracht wird, wobei nur Biozide mit einer durchschnittlichen Tröpfchengröße von kleiner 10 µm den Kamin überwinden können und größere Tröpfchen, abhängig von Strömungsgeschwindigkeit und Kaminhöhe schwerkraftbedingt in der Vorrichtung verbleiben und erneut vernebelt werden.

Eine Vorrichtung zum Ausbringen von wässrigen Bioziden, mit polymerhaltigen Wirkstoffen, in Form von Nebel, mit einer durchschnittlichen Tröpfchengröße von kleiner 10 µm in einen Raum mittels Ultraschalltechnologie, insbesondere zur Desinfektion des Raumes umfasst einen das wässrige Biozid aufnehmenden Behälter, zumindest eine das wässrige Biozid zerstäubende Ultraschallmembrane und mindestens ein Gebläse, welches das zerstäubte wässrige Biozid in den Raum ausbringt.

Der Behälter weist insbesondere einen eine maximale Füllhöhe des wässrigen Biozids über der wenigstens einen Membrane begrenzenden Überlauf, einen Vorratsbehälter für das wässrige Biozid und eine Fördereinrichtung zum Einbringen von wässrigem Biozid aus dem Vorratsbehälter in den Behälter auf. Der Überlauf mündet wiederum im Vorratsbehälter. Damit können die Vernebelungsleistung und die durchschnittliche Tröpfchengröße während wenigstens eines Behandlungszyklusses zumindest annähernd konstant gehalten werden.

Dem Behälter kann, vorzugsweise je über einer Ultraschallmembrane, wenigstens ein, einen Behälterauslass für vernebeltes, wässriges Biozid bildender, Kamin zugehören, um zu verhindern, dass zu große Tröpfchen die Vorrichtung verlassen.

Um gegebenenfalls gezielte Bereiche, Flächen od. dgl. desinfizieren und/oder desodorieren zu können, empfiehlt es sich, wenn dem Behälter ein, einen Behälterauslass für vernebeltes, wässriges Biozid bildender, an seinem Ende gegebenenfalls mit einer Düse ausgestatteter, Schlauch zugehört.

Die Erfindung wird anhand eines Ausführungsbeispiels näher erläutert, wobei das gewählte Beispiel eine Desinfektion eines Fahrzeuginnenraumes und der Klimaanlage vorsieht. Als Desinfektions- bzw. Desodorierungsmittel kommen wässrige Biozide mit polymerhältigen Wirkstoffen wie beispielsweise CAS 374572-91-5, CAS 57028-96-3, CAS 27083-27-8, CAS 32289-58-0 (PHMB) od. dgl. zum Einsatz. So wird die erfindungsgemäße Vorrichtung zur Anwendung des erfindungsgemäßen Verfahrens im Fahrzeuginnenraum, installiert. Die Vorrichtung mit dem Biozid im Behälter erwärmt dieses gegebenenfalls und die Membran beginnt wenige Zentimeter unter der Fluidoberfläche zu schwingen, sodass ein Ausbringen der Wirkstoffe wunschgemäß erfolgt. Die Lösung wird zusätzlich durch zumindest einen Ventilator im Aufsteigen aus der Vorrichtung unterstützt.

Zur Desinfektion der Klimaanlage im Fahrzeug wird diese in mittlerer Stellung aktiviert, wobei sämtliche Kanäle der Klimaanlage durchgeschalten werden.

Nach mehreren Minuten wird in diesem Beispiel die Klimaanlage wieder abgeschalten, sodass sich die weiterhin ausgebrachte Lösung homogen in der Fahrzeugkabine verteilt und dabei die Oberflächen benetzt. Bei ausreichend hoher Wirkstoffdichte im Fahrzeug wird die Vorrichtung abgeschalten. Nach entsprechender Wirkdauer wird über die Klimaanlage und durch Öffnen der Fahrzeugtüren und bzw. oder der Fenster das Fahrzeug ausreichend durchlüftet. Die Vorrichtung wird wieder entfernt.

### Ausführungsbeispiel der Erfindung

Eine Vorrichtung zum Ausbringen von wässrigen Bioziden 2, mit polymerhaltigen Wirkstoffen, in Form von Nebel, mit einer durchschnittlichen Tröpfchengröße von kleiner 10 µm in einen Raum mittels Ultraschalltechnologie, insbesondere zur Desinfektion des Raumes, umfasst unter anderem einen das wässrige Biozid aufnehmenden Behälter, zwei das wässrige Biozid zerstäubende Ultraschallmembranen und ein Gebläse, welches das zerstäubte wässrige Biozid, den Nebel, in den Raum ausbringt. Behälter, Ultraschallmembranen und Gebläse sind in einem gemeinsamen Gehäuse angeordnet.

Der Behälter weist einen eine maximale Füllhöhe des wässrigen Biozids begrenzenden Überlauf auf, der über eine Leitung in einem Vorratsbehälter für das wässrige Biozid mündet. Das wässrige Biozid aus dem Vorratsbehälter wird mit einer Fördereinrichtung über eine Versorgungsleitung in den Behälter eingebracht. Die Förderleistung ist von einer Steuerung dabei so vorzugeben, dass wenigstens die vernebelte Biozidmenge zu jedem Zeitpunkt ersetzt wird. Ein Umwälzen des Biozides durch die Vorrichtung ist erwünscht. Zum Temperieren des Biozides sind eine Heizung und ein Temperatursensor im Vorratsbehälter vorgesehen.

Dem Behälter gehört über jeder Ultraschallmembrane ein einen Behälterauslass für vernebeltes, wässriges Biozid bildender, Kamin zu. Das zerstäubte Biozid wird mit Hilfe eines weiteren Gebläses im Raum verteilt.

## Patentansprüche

1. Verfahren zum Ausbringen eines wässrigen, polymeren, Guanidin enthaltenden Biozids in Form von Nebel mit einer durchschnittlichen Tröpfchengröße von kleiner 10 µm in einen Raum mittels Ultraschalltechnologie zur Desinfektion des Raumes, wobei das Biozid zunächst mit wenigstens einer wenige Zentimeter unter der Fluidoberfläche schwingenden Ultraschallmembrane zerstäubt wird, wonach das zerstäubte, wässrige polymere Desinfektionsmittel mit Luft als Trägermedium in den Raum ausgebracht wird.

2. Verfahren nach Anspruch 1, **dadurch gekennzeichnet, dass** das zerstäubte Biozid mit Luft als Trägermedium, mit mindestens einem Gebläse, in den Raum ausgebracht wird.

3. Verfahren nach Anspruch 1 oder 2, **dadurch gekennzeichnet, dass** ein Guanidin enthaltendes polymeres Biozid mit der Ultraschallmembrane, mit einer Frequenz von >1 MHz zerstäubt wird.

4. Verfahren nach einem der Ansprüche 1 bis 3, **dadurch gekennzeichnet, dass** das Biozid mit wenigstens einer Ultraschallmembrane aus Kunststoff, Keramik, Quarz oder Metall zerstäubt wird.

5. Verfahren nach einem der Ansprüche 1 bis 4, **dadurch gekennzeichnet, dass** das flüssige Biozid vor oder nach der Zerstäubung erwärmt wird.

6. Verfahren nach einem der Ansprüche 1 bis 5, **dadurch gekennzeichnet, dass** das zerstäubte Biozid mit Hilfe von zumindest einem weiteren Gebläse im Raum verteilt wird.

7. Verfahren nach einem der Ansprüche 1 bis 6, **dadurch gekennzeichnet, dass** das Biozid aus einem Vorratsbehälter derart durch einen wenigstens eine Ultraschallmembrane aufweisenden Behälter mit einer Fördereinrichtung umgewälzt wird, dass der Pegel des wässrigen Biozids über der Membrane konstant gehalten wird.

8. Verfahren nach einem der Ansprüche 2 bis 7, **dadurch gekennzeichnet, dass** das vernebelte Biozid mit dem Gebläse über einen Kamin ausgebracht wird, wobei nur Biozide mit einer durchschnittlichen Tröpfchengrö0e von kleiner 10 µm den Kamin überwinden.

## Claims

1. Method for dispensing an aqueous, polymeric guanidine-containing biocide in the form of mist with an average droplet size of less than 10 µm into a room by means of ultrasonic technology for disinfecting the room, wherein the biocide is initially atomised using at least one ultrasonic membrane oscillating a few centimetres below the fluid surface, after which the atomised, aqueous polymeric disinfectant is dispensed into the room with air as the carrier medium.

2. Method as claimed in claim 1, **characterised in that** the atomised biocide is dispensed into the room with air as the carrier medium, using at least one fan.

3. Method as claimed in claim 1 or 2, **characterised in that** a guanidine-containing polymeric biocide is atomised using the ultrasonic membrane at a frequency of >1 MHz

4. Method as claimed in any one of claims 1 to 3, **characterised in that** the biocide is atomised using at least one ultrasonic membrane consisting of synthetic material, ceramic, quartz or metal.

5. Method as claimed in any one of claims 1 to 4, **characterised in that** the liquid biocide is heated before or after the atomisation.

6. Method as claimed in any one of claims 1 to 5, **characterised in that** the atomised biocide is distributed in the room with the aid of at least one further fan.

7. Method as claimed in any one of claims 1 to 6, **characterised in that** the biocide is circulated from a storage container through a container having at least one ultrasonic membrane using a conveying device in such a way that the level of the aqueous biocide above the membrane is kept constant.

8. Method as claimed in any one of claims 2 to 7, **characterised in that** the nebulised biocide is dispensed using the fan via a chimney, wherein only biocides having an average droplet size of less than 10 µm rise out of the chimney.

## Revendications

1. Procédé pour disséminer un biocide polymère aqueux contenant de la guanidine sous la forme de brouillard avec une taille moyenne de gouttelettes de moins de 10 µm dans une pièce au moyen d'une technologie par ultrasons pour désinfecter la pièce, selon lequel le biocide est d'abord vaporisé avec au moins une membrane à ultrasons vibrant quelques centimètres sous la surface du fluide, après quoi le désinfectant polymère aqueux vaporisé est disséminé dans la pièce avec de l'air comme agent porteur.

2. Procédé selon la revendication 1, **caractérisé en ce que** le biocide vaporisé avec de l'air comme agent porteur est disséminé dans la pièce avec au moins un ventilateur.

3. Procédé selon la revendication 1 ou 2, **caractérisé en ce qu'**un biocide polymère contenant de la guanidine est vaporisé avec la membrane à ultrasons à une fréquence supérieure à 1 MHz.

4. Procédé selon une des revendications 1 à 3, **caractérisé en ce que** le biocide est vaporisé avec au moins une membrane à ultrasons en matière plastique, en céramique, en quartz ou en métal.

5. Procédé selon une des revendications 1 à 4, **caractérisé en ce que** le biocide liquide est chauffé avant ou après la vaporisation.

6. Procédé selon une des revendications 1 à 5, **caractérisé en ce que** le biocide vaporisé est réparti dans la pièce à l'aide d'au moins un autre ventilateur.

7. Procédé selon une des revendications 1 à 6, **caractérisé en ce que** le biocide sortant d'un récipient de stockage est mis en circulation à travers un récipient présentant au moins une membrane à ultrasons avec un dispositif de transport de telle sorte que le niveau du biocide aqueux au-dessus de la membrane est maintenu constant.

8. Procédé selon une des revendications 2 à 7, **caractérisé en ce que** le biocide pulvérisé est disséminé avec le ventilateur par une cheminée, selon lequel seuls les biocides avec une taille moyenne de gouttelettes de moins de 10 µm franchissent la cheminée.
